# EUROPÄISCHE PATENTSCHRIFT

(11) **EP 3 202 434 B1**
(45) Veröffentlichungstag und Bekanntmachung des Hinweises auf die Patenterteilung: **06.10.2021**
(21) Anmeldenummer: 17154624.5
(22) Anmeldetag: 03.02.2017
(51) Int. Cl.: A61M 1/16

(54) **FILTERMODUL-VERPACKUNGS-EINHEIT**
FILTER MODULE PACKAGING UNIT
UNITÉ D'EMBALLAGE DE MODULE DE FILTRE

(30) Priorität: 05.02.2016 DE 102016102087
(43) Veröffentlichungstag der Anmeldung: 09.08.2017
(73) Patentinhaber: B. Braun Avitum AG, 34212 Melsungen (DE)
(72) Erfinder: DÖRING, Stefan, 01326 Dresden (DE)
(74) Vertreter: Winter, Brandl - Partnerschaft mbB

(56) Entgegenhaltungen:
- DE-A1- 1 486 518
- DE-A1-102007 021 137
- DE-A1-102007 036 734
- US-A1- 2005 063 859
- US-A1- 2016 015 457

## Beschreibung

Die Erfindung betrifft eine sterile Filtermodul-/Dialysatorverpackungseinheit, aufweisend ein in einer Primärverpackung steril verpacktes Filtermodul/einen Dialysator (Filterpatrone, Reinigungsfilter für Blutbehandlungsmaschinen, etc.).

Bei der Herstellung steriler Medizinprodukte, insbesondere Filtermodule/Dialysatoren, ist sicherzustellen, dass das Produkt bis zur Anwendung an einem Patienten oder Verwendung im Rahmen einer Behandlung steril bleibt. Dazu ist zu gewährleisten, dass entweder die Sterilbarriere an das Produkt gelegt wird oder dass die Verpackung eine Sterilbarriere gegenüber der Umgebung ausbildet, die unter Annahme realistischer Lagerbedingungen die auf dem Produkt angegebene Haltbarkeitsdauer (Shelflifetime) über unverletzt bleibt.

Filtermodule/Dialysatoren sind in ihrer äußeren Form ausschließlich im Hinblick auf fertigungs- und anwendungstechnische Erfordernisse ausgelegt. Es liegt auf der Hand, dass sich aus dieser Form besondere Anforderungen an die Filtermodul-/Dialysatorverpackung ableiten. Problematisch hinsichtlich der Verpackung sind insbesondere am Filtermodul/Dialysator standardisierte, abstehende und scharfkantige Konnektoren sowie Kanten am Filtermodul/Dialysator und an Schutzkappen (Protection Caps) des Filtermoduls/ Dialysators.

Bekannte Verpackungen für Medizinprodukte, insbesondere für Filtermodule/Dialysatoren, bestehen primär aus einem Kunststoff- oder Aluminium-Schlauch- oder einem Siegelrandbeutel (als Primärverpackung) sowie aus einem Tray aus Kunststoff, Pappe oder Faserguss und ggf. einem Umkarton (als Sekundärverpackung). Insbesondere das Tray weist meist eine Form auf, die im Wesentlichen der Form des in der Primärverpackung verpackten Produkts entspricht, so dass eine Art Formschluss gegeben ist, mittels dem eine lagesichere Verpackung erstrebt wird.

Einige Medizinprodukte, insbesondere Filtermodule/Dialysatoren, müssen ggf. unter sauerstofffreien Bedingungen sterilisiert werden. Das bedeutet, dass zum Zeitpunkt der Sterilisierung das Innere der Primärverpackung absolut sauerstofffrei sein muss. Dies wird in aller Regel durch Absorption des Sauerstoffs durch einen geeigneten Träger, einen sogenannten Getter, realisiert. Das Trägermaterial kann dabei beispielsweise Eisenpulver oder ein Polymer sein. Der Absorber kann als sogenanntes Sachet der Primärverpackung zugeführt werden oder in der Struktur des Verpackungsmaterials (Folie) enthalten sein.

Es ist ein beträchtlicher Nachteil, dass eine Bindung von molekularem Sauerstoff im abgeschlossenen System der Primärverpackung eine Volumenreduzierung bzw. einen Unterdruck (bei einer formunveränderlichen Umgebung) zur Folge hat. Bekannte Verpackungssysteme sind nicht formstabil, folglich reduziert sich ihr Volumen entsprechend der Sauerstoffbindung nach Verschließen der Verpackung in nicht kontrollierbarer Weise. Eine solche Volumenreduzierung der Primärverpackungen ermöglicht Relativbewegungen zwischen verpackten Filtermodulen/Dialysatoren untereinander innerhalb der Sekundärverpackung sowie zwischen verpackten Filtermodulen/Dialysatoren und der Sekundärverpackung, wobei diese Relativbewegungen wiederum zu Beschädigungen der Sterilbarriere führen können.

Bei bekannten Filtermodul-/Dialysatorverpackungen wird der vorstehend beschriebenen Problematik von Relativbewegungen infolge einer Volumenreduzierung bei Sterilisierung und eine daraus folgende mögliche Schädigung der Sterilbarriere durch Einsatz entsprechend dicker Folien und/oder einer sauerstoffreduzierten Atmosphäre während eines Verpackungsprozesses entgegengewirkt. Beide Vorgehensweisen bewirken in nachteiliger Weise hohe Material- bzw. Verfahrenskosten. Ein weiterer Nachteil ist, dass Volumenreduzierungen durch Vorsehen einer sauerstoffreduzierten Atmosphäre im Verpackungsprozess nicht vollständig ausgeschlossen werden können, da in einer derartigen Verpackung, insbesondere in einer Filtermodul-/Dialysatorverpackung 100%-ige Sauerstofffreiheit gewährleistet sein muss. In der Folge sind ein Einsatz eines Absorbers und daraus folgende Volumenreduzierungen unumgänglich. Dickere, mechanisch belastbarere Verpackungsmaterialien für die Primärverpackung erhöhen die Produktkosten und gewähren dennoch keine 100%-ige Sicherheit für die Unversehrtheit der Sterilbarriere.

US 2005/0063859 A1 zeigt eine Container-Verpackung für eine Blutbehandlungsvorrichtung mit Hohlfasern. Die Blutbehandlungsvorrichtung wird vor dem Sterilisationsprozess in den Container, der einen Getter zur Sauerstoffabsorption aufweist, eingelegt und dann verschlossen.

DE 10 2007 021 137 A1 zeigt eine Verpackung für Lebensmittel mit einer Schale, in die das Lebensmittel eingelegt wird, und einer Folie, die über die Schale und das Lebensmittel gelegt, angesaugt und an den Rändern der Schale versiegelt wird, wobei das Packgut durch Formschluss fixiert wird.

US 2016/0015457 A1 zeigt ein nestbares Tray zur Aufnahme von Spritzen, die in dem Tray formschlüssig gehalten werden können.

Ausgehend von dem vorstehend beschriebenen Stand der Technik liegt der vorliegenden Erfindung die Aufgabe zugrunde, die zuvor angeführten Nachteile zu beseitigen, insbesondere eine sterile Filtermodul-/Dialysatorverpackung bereit zu stellen, mittels der Beschädigungen der Sterilbarriere infolge von Relativbewegungen zwischen Verpackungen, die durch unkontrollierte Volumenreduzierung möglich werden, minimiert oder vorzugsweise verhindert werden können. Die Verpackung selbst soll vorzugsweise gegenüber Volumenreduzierung unempfindlich sowie kostengünstig und formstabil sein.

Nach der Erfindung wird diese Aufgabe gelöst durch eine sterile Filtermodul-/ Dialysatorverpackungseinheit mit den Merkmalen des Anspruchs 1. Konkret wird eine sterile Filtermodul-/ Dialysatorverpackungseinheit bereitgestellt, aufweisend ein in einer Primärverpackung steril verpacktes Filtermodul/einen Dialysator mit einem im Wesentlichen zylinderförmigen Mittenabschnitt und einem endseitig des Mittenabschnitts ausgebildeten Filtermodul-/Dialysatoranschluss, vorzugsweise mit jeweils einem endseitig des Mittenabschnitts ausgebildeten Filtermodul-/ Dialysatoranschluss, wobei die Primärverpackung eine Blisterverpackung ist mit einem eine Aufnahme für das Filtermodul/den Dialysator ausbildenden Kunststoffformteil als Unterteil und einer mit diesem einen Aufnahmeraum für das Filtermodul/den Dialysator ausbildenden Oberfolie, wobei die Oberfolie hermetisch dicht am Unterteil festgelegt (Schweißen, Kleben) ist und das Filtermodul/den Dialysator zumindest abschnittsweise durch Formschluss fixiert, insbesondere gegenüber dem Unterteil oder zwischen sich und dem Unterteil fixiert. Man kann auch sagen, dass die Erfindung einen Hart-Weich-Blister als (Primär-)Verpackung für Filtermodule/Dialysatoren bereitstellt. Die Oberfolie der Primärverpackung schmiegt sich zumindest an den Mittenabschnitt des Filtermoduls/Dialysators eng an (liegt an). Zwischen dem Unterteil und der Oberfolie ist daher nur eine geringe Menge an Luft und damit molekularem Sauerstoff enthalten, was für eine Sterilisierung des in der Primärverpackung verpackten Filtermoduls/Dialysators vorteilhaft ist. Im Falle einer Sterilisierung mittels z.B. Gamma-Strahlung, wofür in der verschlossenen und abgedichteten Verpackung enthaltener molekularer Sauerstoff zu entfernen ist, fällt eine Volumenreduzierung infolgedessen in vorteilhafter Weise gering aus.

Die Verpackung eines Filtermoduls/Dialysators macht einen wesentlichen Bestandteil seiner Herstellungskosten aus. Durch die Erfindung kann in vorteilhafter Weise eine Senkung von Kosten erzielt werden, da die einzelnen Bestandteile besonders einfach ausgebildet sein können und dennoch eine ausreichende Stabilität der (Primär)Verpackung bei einer Volumenreduzierung zum Beispiel infolge von Sauerstoffabsorption sicherstellen. Die Hart-Weich-Blisterverpackung (Primärverpackung) nach der Erfindung ist besonders einfach und gut an die Form des verpackten Filtermoduls/Dialysators anzupassen oder anzunähern. Relativbewegungen zwischen dem Filtermodul/Dialysator und der Primärverpackung können auf ein Mindestmaß reduziert oder gar eliminiert werden, da das in der Primärverpackung verpackte Filtermodul/der Dialysator durch Formschluss mit der Oberfolie oder durch Formschluss mit der Oberfolie und dem Unterteil sicher und fest fixiert ist. Da das verpackte Filtermodul/der Dialysator hauptsächlich durch die Oberfolie gehalten ist, kann das Unterteil einfach ausgestaltet sein, was eine einfache und kostengünstige Verpackung ermöglicht. Zudem ist es dadurch möglich, ein standardisiertes Unterteil für verschiedene Filtermodul-/ Dialysatorgrößen mit unterschiedlichen Durchmessern zu verwenden. Weiterhin kann die Kapazität des Sauerstoffabsorbers bzw. Getters aufgrund des geringeren Verpackungsvolumens reduziert werden, so dass eine weitere Senkung der Herstellkosten erreichbar ist.

Im Sinne der Erfindung wird eine Verpackung mit einem Hart-Weich-Blister für Filtermodule/Dialysatoren (oder auch anderen Medizinprodukte) genutzt. Dieser ist derart ausgebildet, dass das Filtermodul/der Dialysator in der Primärverpackung sicher und fixiert gehalten oder lagepositioniert ist. Nach einer Ausführungsform kann das (nämlich die Lagepositionierung) durch Definition eines Bereiches innerhalb der Verpackung erfolgen, an dem die Verpackung, insbesondere das Formteil sich infolge einer Volumenreduzierung ohne Beeinträchtigung der Gesamtstabilität und der grundsätzlichen Form der Verpackung verformen kann. Ohne Beeinträchtigung der grundsätzlichen Form der Verpackung bedeutet in diesem Sinne, dass bestimmte Außenbereiche der Verpackung, über die sie zum Beispiel an anderen Primärverpackungen oder an einer Umverpackung abgestützt ist, sich nicht verformen (Einrichten von Sollverformungsbereichen). Ebenfalls im Wesentlichen unverformt bleiben Innenabschnitte der Verpackung, insbesondere des Formteils, über die oder an denen der Dialysator/Filtermodul gehalten, gelagert oder abgestützt ist.

Vorteilhafte Ausführungsformen der Erfindung sind in den Unteransprüchen beansprucht und werden nachfolgend näher erläutert.

Um eine Strahlen(Gamma)-Sterilisierung eines in der erfindungsgemäßen Verpackung verpackten Filtermoduls/Dialysators zu ermöglichen, weist die Filtermodul-/ Dialysatorverpackung bei einer Ausführungsform einen Getter zur Bindung von in der Primärverpackung, insbesondere in dem Aufnahmeraum vorliegendem molekularen Sauerstoff auf. Eine Volumenreduzierung oder ein im Aufnahmeraum entstehender Unterdruck infolge einer Bindung von Sauerstoff durch den Getter bewirkt keine Relativbewegungen zwischen einzeln verpackte Filtermodulen/Dialysatoren, da sich die Primärverpackung, insbesondere die Oberfolie, erfindungsgemäß bereits vor der Sauerstoffabsorption an das verpackte Filtermodul/den Dialysator, anschmiegt. Eine weitere Volumenreduzierung infolge Sauerstoffbindung führt dann in besonders vorteilhafter Weise zu einem noch festeren Anschmiegen der Verpackung an das Filtermodul/den Dialysator. Man kann also sagen, dass die bei Verpackungen nach dem Stand der Technik in der Regel problematische Volumenverringerung infolge einer Bindung von molekularem Sauerstoff im Rahmen der Erfindung in vorteilhafter Weise ausgenutzt wird, um eine noch bessere Lagerung/Fixierung des Filtermoduls/Dialysators in der Primärverpackung zu bewirken. Somit werden durch die Erfindung nicht nur Relativbewegungen zwischen Filtermodul/Dialysator(Primär)Verpackungen erfolgreich unterdrückt, sondern es wird auch das verpackte Filtermodul/der Dialysator besonders gut lagefixiert.

Eine Besonderheit der Erfindung ist, dass das Unterteil vorzugsweise so gestaltet ist, dass es das Filtermodul/den Dialysator sicher aufnimmt. Vorzugsweise ist das Unterteil als eine Art Tablett ausgebildet, das eine Vertiefung für das Filtermodul/den Dialysator besitzt, dessen Höhe jedoch (deutlich) geringer als die Abmessungen des Filtermoduls/Dialysators quer zu dessen Axialrichtung ist. Vorzugsweise beträgt die Tiefe der im Unterteil ausgestalteten Aufnahme für das Filtermodul/den Dialysator weniger als die Hälfte des Durchmessers des Filtermoduls/Dialysators, besonders bevorzugt weniger als ein Drittel des Durchmessers des Filtermoduls/Dialysators, noch bevorzugter weniger als ein Viertel des Durchmessers des Filtermoduls/Dialysators. Auf diese Weise wird einfach sichergestellt, dass das Filtermodul/der Dialysator zum größeren Teil von der Oberfolie umhüllt und durch diese lagefixiert ist. Des Weiteren ist das Unterteil derart gestaltet, dass ein Verschließen von Filtermodul-/ Dialysatoranschlüssen durch die Oberfolie verhindert wird bzw. nicht möglich ist. Diese müssen im Falle einer Strahlen(Gamma)-Sterilisierung zwingend freibleiben, um eine Bewegung von Sauerstoff-Molekülen aus dem Faserbündel des Filtermoduls/Dialysators zu ermöglichen. Insbesondere kann die Oberfolie (eng) an dem zylinderförmigen Mittenabschnitt des Filtermoduls/Dialysators anliegen und die jeweils endseitig des Mittenabschnitts ausgebildeten Filtermodul-/Dialysatoranschlüsse des Filtermoduls/Dialysators nicht kontaktieren. Vorzugsweise ist die Oberfolie eine Schrumpffolie.

Das feste Unterteil der Blisterkombination kann dabei einen Rand aufweisen. Die Oberfolie kann an dem vorzugsweise kontinuierlich umlaufenden Rand des Unterteils aufgesiegelt sein. Der Rand kann insbesondere formschlüssig zum Umkarton sein und somit Relativbewegungen zwischen Primärverpackungen und Sekundärverpackung/Umkarton einschränken bzw. verhindern.

Das Unterteil bildet vorzugsweise beiderseits endseitig des Mittenabschnitts des Filtermoduls/Dialysators jeweils eine gegenüber der Außenkontur des Filtermoduls/Dialysators, insbesondere gegenüber den Filtermodul/Dialysatoranschlüssen, aufgeweitete Anschlussaufnahme aus. In diese können die Filtermodul-/Dialysatoranschlüsse aufgenommen sein, so dass diese in vorteilhafter Weise weder durch das Unterteil noch durch die Oberfolie verschlossen sind und in der jeweiligen Anschlussaufnahme offen und strömungstechnisch mit dem Aufnahmeraum, insbesondere mit dem Anschlussaufnahmevolumen, verbunden sind.

Der erfindungsgemäße Hart-Weich-Blister entfaltet seine Vorteile in Kombination mit einem entsprechend geformten Tray in der Sekundärverpackung. Bei einer Ausführungsform der Erfindung weist die Filtermodul-/Dialysatorverpackungseinheit daher ein Tray auf. Dieses weist vorzugsweise eine Auflage oder Aufnahme für den von der Oberfolie umformten Mittenabschnitt des Filtermoduls/Dialysators auf. Das Tray ist vorzugsweise derart ausgebildet, dass mit dem von der Oberfolie umformten Mittenabschnitt Formschluss in axialer Richtung und/oder in tangentialer Richtung (des Filtermoduls/Dialysators) besteht. Man kann auch sagen, dass das Tray nur zur Auflage des zylindrischen Teils des Filtermoduls/Dialysators dient, die geometrisch kritischen Bereiche des Filtermoduls/Dialysators, die sogenannten "Filtermodul-/Dialysatorköpfe" liegen frei. Das Tray besitzt vorzugsweise eine im Wesentlichen U-förmige Querschnittsform. Es kann insbesondere eine Oberplatte und sich beiderseits daran anschließende Seitenplatten aufweisen, welche als Standbeine fungieren. Insbesondere kann die Oberplatte Vertiefungen, vorzugsweise teilzylinderförmige Vertiefungen, aufweisen, die von der einen Seitenplatte zur gegenüberliegenden Seitenplatte verlaufen, also quer zur axialen Richtung des in der Primärverpackung angeordneten Filtermoduls/Dialysators ausgerichtet sind. Jeweils eine Primärverpackung mit darin verpacktem Filtermodul/Dialysator ist formschlüssig in eine Vertiefung einlegbar bzw. eingelegt. Man kann auch sagen, dass in der Primärverpackung verpackte Filtermodule/Dialysatoren im zylindrischen Teil auf dem Tray liegen, so dass die "Problemzonen" frei auskragen und damit Relativbewegungen ausgeschlossen sind. Die belegten Trays können in einen herkömmlichen Umkarton (Sekundärverpackung) gepackt werden.

Die Filtermodul-/ Dialysatorverpackung kann nach einer weiteren Ausführungsform des Weiteren eine Umverpackung oder Sekundärverpackung aufweisen, in der eine Mehrzahl von Primärverpackungen mit jeweils zumindest einem darin aufgenommenen Filtermodul/Dialysator angeordnet sind. Die Umverpackung ist vorzugsweise derart ausgebildet, dass zwischen dem umlaufenden Rand des Unterteils einer darin angeordneten Primärverpackung und der Umverpackung zumindest abschnittsweise Formschluss vorliegt. Vorzugsweise ist das Tray formschlüssig in die Umverpackung eingelegt bzw. einlegbar.

Zusammenfassend kann man sagen, dass die Grundidee, auf der die Erfindung beruht, ist, einen Hart-Weich-Blister für eine sterile Verpackung eines Filtermoduls/Dialysators zu verwenden. Zumindest im zylindrischen Teil der Filtermodule/Dialysatoren gemäß vorstehender Definition schließt sich die Primärverpackung eng an das verpackte Filtermodul/den Dialysator an. Ein korrespondierender, formähnlicher Tray passt zur und unterstützt das Filtermodul/den Dialysator im Karton exakt an dieser Stelle, so dass konstruktiv empfindliche Stellen des Filtermoduls/Dialysators (Protection Caps und Konnektoren) frei, d.h. ohne Berührung zur Primärverpackung, zur Sekundärverpackung und/oder einer benachbarten Verpackungseinheit liegen. Die infolge der eng am Filtermodul/Dialysator anliegenden Oberfolie geringe Luftmenge in der (Primär)Verpackung reduziert eine im Falle einer Strahlen(Gamma)-Sterilisation erforderliche Absorberkapazität. Zusätzlich schränkt die enganliegende Folie Relativbewegungen zwischen dem Filtermodul/Dialysator und der Folie ein und ermöglicht einen Formschluss zu einem korrespondierenden Tray. Relativbewegungen zwischen Primärverpackungen untereinander sowie zwischen Primärverpackungen und der Sekundärverpackung sind damit ausgeschlossen, einer Verletzung der Sterilbarriere wird somit wirkungsvoll entgegengewirkt.

Mit der Erfindung werden unter anderem die folgenden Vorteile erzielt:
- Verbesserte Möglichkeit der Automatisierung (Handling), was insbesondere zu einer Senkung von Herstellkosten und damit zur Reduzierung der Verpackungskosten führt,
- Ermöglichung einer hohe Anzahl von Filtermodulen/Dialysatoren im Umkarton, was insbesondere zu einer Senkung von Logistikkosten führt,
- reduzierte Formveränderung bei Bindung molekularen Sauerstoffs in der Primärverpackung aufgrund reduzierter Luftmenge in der Verpackung,
- neue Designmöglichkeiten durch gerade "Kommunikationsflächen" zwischen einzelnen Primärverpackungen sowie einer Sekundärverpackung,
- Einschränkung von diversen Relativbewegungen, die in der Regel zu Verletzungen der Verpackung und der Sterilbarrieren führen, durch die eng am Filtermodul/Dialysator anliegende Oberfolie
- Reduzierung der benötigten Absorberkapazität und
- höhere Produktsicherheit durch deutlich erkennbare Undichtigkeiten, wenn die Oberfolie nicht mehr eng am Filtermodul/Dialysator anliegt.

Die Erfindung wird im Folgenden anhand beispielhafter, nicht einschränkender und in den angehängten Figuren gezeigter Ausführungsformen näher erläutert. Dabei zeigt:
Figur 1 eine schematische perspektivische Darstellung einer sterilen Dialysator-Primärverpackung in einer ersten Ausführungsform,
Figur 2 die Dialysator-Primärverpackung der Figur 1 in einer Schnittansicht quer zur Axialrichtung,
Figur 3 eine perspektivische Ansicht eines Tray zur Verwendung im Rahmen der Erfindung,
Figur 4 eine Umverpackung zur Verwendung im Rahmen der Erfindung.

Im Anschluss wird vornehmlich der Begriff "Dialysator" verwendet, wobei an dieser Stelle darauf hingewiesen wird, dass damit auch jedwede Art von Filtermodul zu verstehen ist.

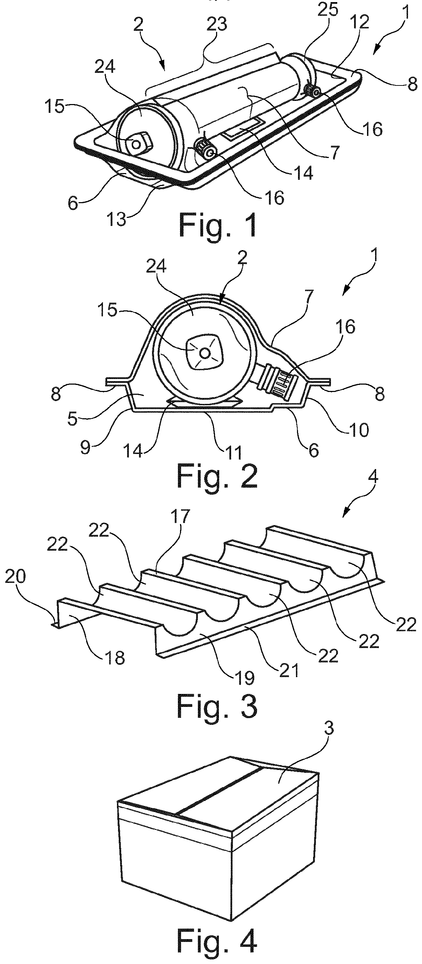

Eine sterile Dialysator-Primärverpackung 1 (nachfolgend auch als Primärverpackung 1 bezeichnet) ist in Figur 1 mit einem steril darin verpackten Dialysator 2 gezeigt. Die Primärverpackung 1 wird zusammen mit weiteren Primärverpackungen 1 mit darin verpackten Dialysatoren mittels einer Sekundärverpackung oder Umverpackung/Umkarton 3 und einem oder mehreren Trays 4 zu einer Verpackungseinheit kombiniert. Bei der Sekundärverpackung 3 handelt es sich zum Beispiel, wie in den Figur 4 gezeigt ist, um einen Faltkarton 3.

Die Primärverpackung 1 ist nach der Erfindung als Hart-Weich-Blisterverpackung mit einem eine Aufnahme oder Aufnahmewanne 5 für den Dialysator 2 ausbildenden Unterteil 6 realisiert. Nach bestimmungsgemäßer Anordnung des Dialysators 2 darin ist das Unterteil 6 hermetisch mit einem daran angeordneten Deckelteil 7 in Form einer Oberfolie 7 verschlossen. Die Oberfolie 7 ist eine Kunststofffolie, vorzugsweise eine Schrumpffolie, die auf einem peripheren umlaufenden Rand 8 des Unterteils 6 angeordnet und mit diesem hermetisch dicht versiegelt ist.

Das Unterteil 6 ist aus Kunststoff ausgebildet, beispielsweise durch Molding, und besitzt eine definierte Form mit zwei einander gegenüberliegenden stabilen Seitenwänden 9, 10 und einer zwischen diesen angeordneten Wanne 11. Die Stirnseiten 12, 13 des Unterteils bilden zusammen mit den Seitenwänden 9, 10 und der Wanne 11 die Aufnahme 5 für den Dialysator 2 aus und gehen an deren offener Seite in dem umlaufenden Rand 8 über. Wanne 11, Seitenwände 9, 10 und Stirnwände 12, 13 sind mit entsprechender Stabilität ausgebildet. Unter "stabil" in diesem Sinne ist zu verstehen, dass sich das Unterteil bei einem sich in der Primärverpackung bildenden Unterdruck aufgrund einer Bindung von Sauerstoff im Wesentlichen nicht verformt. Im Gegensatz zum Unterteil 6 weist die Oberfolie eine verminderte Formstabilität auf. Verminderte Formstabilität in diesem Sinne bedeutet, dass sie sich bei einem sich in der Aufnahme 5 bildenden Unterdruck aufgrund einer Bindung von molekularem Sauerstoff zumindest abschnittsweise verformt und einen Druckausgleich herbeiführt. Dabei legt sich die Oberfolie straffer oder enger in später beschriebener Weise an den Dialysator 2 an.

Um molekularen Sauerstoff, der nach dem Verschließen der Primärverpackung 1 in der darin eingeschlossenen Atmosphäre vorliegt, zu binden und für eine Strahlen(Gamma)-Sterilisation des verpackten Dialysators aus der Atmosphäre zu entfernen, ist ein Getter 14 in der Aufnahme 5 platziert.

Die Innenkontur der Aufnahme 5 ist derart ausgebildet, dass ein Bereich des Dialysators 2 darin aufgenommen ist. Die stabilen Seiten 9, 10, 12, 13 des Aufnahmeraums 5 bilden ein Volumen aus, das größer als das Volumen der darin aufgenommenen Abschnitte des Dialysators 2 ist, so dass dessen radiale Dialysatoranschlüsse 15 und axiale Dialysatoranschlüsse 16 nicht von dem Unterteil 6 oder der Oberfolie 7 verschlossen sind (siehe insbesondere in Figur 2). Auf diese Weise ist das Innere des Dialysators 2 strömungstechnisch mit der Atmosphäre der Aufnahme 5 verbunden, so dass auch im Inneren des Dialysators 2 vorliegender molekularer Sauerstoff mittels des Getters 14 gebunden werden kann. Ein zwischen den endseitigen Dialysatoranschlüssen 15, 16 vorliegender mittlerer Zylinderabschnitt 23 des Dialysators 2 ist eng von der Oberfolie 7 umschlossen, siehe insbesondere in Figur 2. Der Dialysator 2 ist auf diese Weise durch die eng am Zylinderabschnitt 23 anliegende Oberfolie 7 in der Aufnahme 5 und damit am Unterteil 6 lagefixiert und gehalten. Man kann auch sagen, dass das Unterteil 5 aus einem der Dialysatorform angepassten Hartteil besteht und die weiche Oberfolie 7 den Dialysator 2 eng umschließt, aber nicht verschließt, so dass der Sauerstoffabsorber 14 wirken und auch molekularen Sauerstoff aus dem Inneren des Dialysators 2 binden kann.

Figur 3 zeigt das Tray 4, das zusammen mit mehreren Primärverpackungen 1 gemäß der Figuren 1 und 2 in einer Umverpackung 3 gemäß der Fig. 4 verpackt wird. Das Tray 4 besitzt in einem Querschnitt quer zur Axialrichtung des Dialysators 2 eine im Wesentlichen u-förmige Gestalt. Es weist eine Oberplatte 17 und sich daran anschließende einander gegenüberliegende Seitenplatten 18, 19 auf. An der von der Oberplatte 17 abgewandten Seite jeder Seitenplatte 18, 19 ist jeweils ein Fuß 20, 21 ausgebildet. In der Oberplatte 17 ist eine Mehrzahl von Vertiefungen 22 ausgebildet. Diese verlaufen vorzugsweise parallel zueinander. Sie sind, wie in Figur 3 gut zu erkennen ist, im Wesentlichen teilzylinderförmig, hier halbzylinderförmig, ausgebildet. Jede der Vertiefungen 22 bildet eine Aufnahme für jeweils einen in einer Primärverpackung 1 verpackten Dialysator 2 aus. Die mit dem darin hermetisch verschlossenen Dialysator 2 versehene Primärverpackung 1 wird so in das Tray 4 gesetzt, dass die Oberfolie 7 zum Tray 4 hinweist. Es besteht ein Formschluss zwischen der jeweiligen Vertiefung 22 und dem Zylinderabschnitt 23 mit der daran eng anliegenden Oberfolie 7. Wie in Figur 1 zu erkennen ist, weisen die beiden endseitigen Abschnitte 24, 25 des Dialysators 2 einen größeren Durchmesser auf als der mittige Zylinderabschnitt 23. Die Breite des Tray 4, also der Abstand der beiden Seitenplatten 18, 19 voneinander, ist derart bemessen, dass eine Anlage zwischen der jeweiligen Seitenplatte 18, 19 und dem auf deren Seite befindlichen Seitenabschnitt 24, 25 des Dialysators 2 vorliegt. Der in der Primärverpackung 1 verpackte Dialysator 2 ist daher durch die jeweilige Vertiefung 22 in tangentialer Richtung und durch die Seitenplatten 18, 19 in axialer Richtung lagefixiert gelagert.

Die Abmessungen des umlaufenden Rands 8 des Unterteils 6 und der Umverpackung 3 sind derart aufeinander abgestimmt, dass eine Mehrzahl von Primärverpackungen 1 lagestabil in der Umverpackung 3 angeordnet werden können.

### Bezugszeichen

- 1: Dialysator-Primärverpackung oder Primärverpackung
- 2: Dialysator
- 3: Sekundärverpackung oder Umverpackung/Umkarton
- 4: Tray
- 5: Aufnahme
- 6: Unterteil
- 7: Oberfolie
- 8: umlaufender Rand
- 9: Seitenwand
- 10: Seitenwand
- 11: Wanne
- 12: Stirnwand
- 13: Stirnwand
- 14: Getter
- 15: Dialysatoranschluss
- 16: Dialysatoranschluss
- 17: Oberplatte
- 18: Seitenplatte
- 19: Seitenplatte
- 20: Fuß
- 21: Fuß
- 22: Vertiefung
- 23: Zylinderabschnitt
- 24: Seitenabschnitt
- 25: Seitenabschnitt

## Patentansprüche

1. Sterile Verpackungseinheit eines medizinischen Filtermoduls, aufweisend mindestens ein in einer Primärverpackung (1) steril verpacktes Filtermodul mit einem im Wesentlichen zylinderförmigen Mittenabschnitt (23) und endseitig des Mittenabschnitts (23) ausgebildeten Filtermodulanschlüssen (15, 16), wobei die Primärverpackung (1) mindestens einen Getter (14) zur Bindung von in der Primärverpackung (1) vorliegendem molekularen Sauerstoff aufweist, **dadurch gekennzeichnet, dass**
die Primärverpackung (1) eine Blisterverpackung mit einem eine Aufnahme (5) für das Filtermodul (2) ausbildenden Kunststoffformteil als hartes Unterteil (6) und einer mit diesem die Aufnahme (5) für das Filtermodul (2) verschließenden weichen Oberfolie (7) ist, wobei die Oberfolie (7) hermetisch dicht am Unterteil (6) festgelegt ist, das Filtermodul (2) zumindest abschnittsweise durch Formschluss fixiert ist und das Unterteil (6) derart gestaltet ist, dass ein Verschließen der Filtermodulanschlüsse (15, 16) durch die Oberfolie verhindert wird.

2. Sterile Verpackungseinheit eines medizinischen Filtermoduls nach Anspruch 1, **dadurch gekennzeichnet, dass** die Oberfolie an dem zylinderförmigen Mittenabschnitt (23) des Filtermoduls (2) anliegt und die jeweils endseitig des Mittenabschnitts (23) ausgebildeten Filtermodulanschlüsse (15, 16) des Filtermoduls (2) nicht kontaktiert, so dass diese offen sind.

3. Sterile Verpackungseinheit eines medizinischen Filtermoduls nach einem der vorstehenden Ansprüche, **dadurch gekennzeichnet, dass** die Oberfolie (7) eine Schrumpffolie ist.

4. Sterile Verpackungseinheit eines medizinischen Filtermoduls nach einem der vorstehenden Ansprüche, **dadurch gekennzeichnet, dass** die Oberfolie (7) an einem Rand (8) des Unterteils (6) aufgesiegelt ist.

5. Sterile Verpackungseinheit eines medizinischen Filtermoduls nach einem der vorstehenden Ansprüche, **dadurch gekennzeichnet, dass** das Unterteil (6) beiderseits endseitig des Mittenabschnitts (23) des Filtermoduls (2) jeweils eine gegenüber der Außenkontur des Filtermoduls (2) aufgeweitete Anschlussaufnahme ausbildet, in der die Filtermodulanschlüsse (15, 16) aufgenommen sind, so dass diese weder durch das Unterteil (6) noch durch die Oberfolie (7) verschlossen sind und in der jeweiligen Anschlussaufnahme offen und strömungstechnisch mit dem Aufnahmeraum (5) verbunden sind.

6. Sterile Verpackungseinheit eines medizinischen Filtermoduls nach einem der vorstehenden Ansprüche, **dadurch gekennzeichnet, dass** die Tiefe der im Unterteil (6) ausgebildeten Aufnahme (5) für das Filtermodul (2) geringer ist als der Durchmesser des zylinderförmigen Mittenabschnitts des Filtermoduls (2).

7. Sterile Verpackungseinheit eines medizinischen Filtermoduls nach einem der vorstehenden Ansprüche, **dadurch gekennzeichnet, dass** diese ein Tray (4) aufweist, das eine Auflage oder Aufnahme für den von der Oberfolie (7) umformten Mittenabschnitt (23) des Filtermoduls (2) aufweist.

8. Sterile Verpackungseinheit eines medizinischen Filtermoduls nach einem der vorstehenden Ansprüche, **dadurch gekennzeichnet, dass** diese eine Umverpackung (3) aufweist, wobei zwischen dem umlaufenden Rand (8) des Unterteils (6) und der Umverpackung (3) zumindest abschnittsweise Formschluss vorliegt.

9. Sterile Verpackungseinheit eines medizinischen Filtermoduls nach einem der Ansprüche 7 oder 8, **dadurch gekennzeichnet, dass** das Tray (4) eine im Wesentlichen u-förmige Querschnittsform mit einer Oberplatte (17) und sich beiderseits daran anschließenden Seitenplatten (18, 19) aufweist, wobei die Oberplatte (17) von der einen Seitenplatte (18) zu gegenüberliegenden Seitenplatte (19) verlaufende Vertiefungen (22) aufweist, in die jeweils eine Primärverpackung (1) mit darin verpacktem Filtermodul (2) formschlüssig eingelegt ist.

10. Sterile Verpackungseinheit eines medizinischen Filtermoduls nach einem der Ansprüche 7 bis 9, **dadurch gekennzeichnet, dass** das Tray (4) oder eine Mehrzahl an Trays (4) zumindest teilweise formschlüssig in die Umverpackung (3) einlegbar bzw. eingelegt ist.

11. Sterile Verpackungseinheit eines medizinischen Filtermoduls nach einem der vorstehenden Ansprüche, **dadurch gekennzeichnet, dass** der Getter (14) durch Absorption des molekularen Sauerstoffs innerhalb der Primärverpackung (1) einen Unterdruck gegenüber dem Atmosphärendruck induziert, wobei das Unterteil (6) im Wesentlichen formstabil ist, so dass ausschließlich die Oberfolie (7) infolge des Unterdrucks deformiert wird.

## Claims

1. Sterile packaging unit of a medical filter module, comprising at least one filter module packaged in a sterile manner in a primary packaging (1), with an essentially cylindrical central section (23) and filter module connections (15, 16) formed at the end of the central section (23), wherein the primary packaging (1) comprises at least one getter (14) to bind molecular oxygen present in the primary packaging (1), **characterized in that**
the primary packaging (1) is a blister packaging with a plastic moulded part as a hard lower section (6) forming a receiving compartment (5) for the filter module (2) and a soft upper foil (7) which, with the lower section (6), closes the receiving compartment (5) for the filter module (2), wherein the upper foil (7) is fixed onto the lower section (6) so as to be hermetically sealed and the filter module (2) is fixed at least in sections by means of form fit, and the lower section (6) is formed such that closing the filter module connections (15, 16) is prevented by the upper foil.

2. Sterile packaging unit of a medical filter module according to claim 1, **characterized in that** the upper foil fits against the cylindrical central section (23) of the filter module (2) and does not come into contact with the filter module connections (15, 16) formed at each end of the central section (23) of the filter module (2), so that they are open.

3. Sterile packaging unit of a medical filter module according to one of the preceding claims, **characterized in that** the upper foil (7) is a shrink foil.

4. Sterile packaging unit of a medical filter module according to one of the preceding claims, **characterized in that** the upper foil (7) is sealed onto an edge (8) of the lower section (6).

5. Sterile packaging unit of a medical filter module according to one of the preceding claims, **characterized in that** the lower section (6) at both ends of the central section (23) of the filter module (2) forms a connection receiving structure, which is widened vis-à-vis the external contour of the filter module (2) and which holds the filter module connections (15, 16) in such a way that these are not closed by the lower section (6) or by the upper foil (7) and are open in the respective connection receiving structure and are fluidically connected with the receiving compartment space (5).

6. Sterile packaging unit of a medical filter module according to one of the preceding claims, **characterized in that** the depth of the receiving compartment (5) for the filter module (2) configured in the lower section (6) is less than the diameter of the cylindrical central section of the filter module (2).

7. Sterile packaging unit of a medical filter module according to one of the preceding claims, **characterized in that** it comprises a tray (4) which comprises a holder or receiving compartment for the central section (23) of the filter module (2) enclosed by the upper foil (7).

8. Sterile packaging unit of a medical filter module according to one of the preceding claims, **characterized in that** it comprises an external packaging (3), wherein there is a form fit at least in sections between the peripheral edge (8) of the lower section (6) and the external packaging (3).

9. Sterile packaging unit of a medical filter module according to one of the claims 7 or 8, **characterized in that** the tray (4) comprises an essentially U-shaped cross-section with an upper plate (17) and side plates (18, 19) joined on both sides of it, wherein the upper plate (17) comprises recesses (22) running from the one side plate (18) to the side plate (19) on the opposite side in which each a primary packaging (1), with a filter module (2) packaged inside it, is inserted in a form fit.

10. Sterile packaging unit of a medical filter module according to one of the claims 7 to 9, **characterized in that** the tray (4) or a number of trays (4) can be or is inserted in the external packaging (3) so as to create at least a partial form fit.

11. Sterile packaging unit of a medical filter module according to one of the preceding claims, **characterized in that** the getter (14) induces negative pressure vis-à-vis the atmospheric pressure by absorption of the molecular oxygen in the primary packaging (1), wherein the lower section (6) is essentially dimensionally stable so that only the upper foil (7) is deformed as a result of the negative pressure.

## Revendications

1. Unité d'emballage stérile d'un module de filtre médical, présentant au moins un module de filtre emballé de manière stérile dans un emballage primaire (1) avec une portion centrale (23) sensiblement cylindrique et des bornes de module de filtre (15, 16) réalisées à l'extrémité de la portion centrale (23), dans laquelle l'emballage primaire (1) présente au moins un getter (14) destiné à fixer l'oxygène moléculaire présent dans l'emballage primaire (1),
**caractérisée en ce que**
l'emballage primaire (1) est un emballage-coque avec une pièce moulée en matière plastique réalisant un logement pour le module de filtre (2) en tant que partie inférieure (6) dure et un film supérieur (7) souple fermant avec celui-ci le logement (5) pour le module de filtre (2), dans lequel le film supérieur (7) est fixé de manière à être fermé hermétiquement au niveau de la partie inférieure (6), le module de filtre (2) est fixé au moins partiellement par complémentarité de formes et la partie inférieure (6) est agencée de sorte qu'une fermeture des bornes de module de filtre (15, 16) est empêchée par le film supérieur.

2. Unité d'emballage stérile d'un module de filtre médical selon la revendication 1, **caractérisée en ce que** le film supérieur repose sur la portion centrale (23) cylindrique du module de filtre (2) et n'entre pas en contact avec les bornes de module de filtre (15, 16) réalisées respectivement à l'extrémité de la portion centrale (23), de sorte que celles-ci sont ouvertes.

3. Unité d'emballage stérile d'un module de filtre médical selon l'une quelconque des revendications précédentes, **caractérisée en ce que** le film supérieur (7) est un film thermorétractable.

4. Unité d'emballage stérile d'un module de filtre médical selon l'une quelconque des revendications précédentes, **caractérisée en ce que** le film supérieur (7) est scellé sur un bord (8) de la partie inférieure (6).

5. Unité d'emballage stérile d'un module de filtre médical selon l'une quelconque des revendications précédentes, **caractérisée en ce que** la partie inférieure (6) réalise des deux côtés à l'extrémité de la portion centrale (23) du module de filtre (2) respectivement un logement de borne élargi par rapport au contour extérieur du module de filtre (2), dans laquelle les bornes de module de filtre (15, 16) sont logées, de sorte que celles-ci ne sont fermées ni par la partie inférieure (6) ni par le film supérieur (7) et dans laquelle chaque logement de borne est relié de manière ouverte et fluidique avec l'espace de logement (5).

6. Unité d'emballage stérile d'un module de filtre médical selon l'une quelconque des revendications précédentes, **caractérisée en ce que** la profondeur du logement (5) réalisé dans la partie inférieure (6) pour le module de filtre (2) est inférieure au diamètre de la portion centrale cylindrique du module de filtre (2).

7. Unité d'emballage stérile d'un module de filtre médical selon l'une quelconque des revendications précédentes, **caractérisée en ce que** celle-ci présente un plateau (4), qui présente un support ou un logement pour la portion centrale (23) du module de filtre (2) transformée par le film supérieur (7).

8. Unité d'emballage stérile d'un module de filtre médical selon l'une quelconque des revendications précédentes, **caractérisée en ce que** celle-ci présente un suremballage (3), dans laquelle une complémentarité de formes est présente au moins partiellement entre le bord périphérique (8) de la partie inférieure (6) et le suremballage (3).

9. Unité d'emballage stérile d'un module de filtre médical selon l'une quelconque des revendications 7 ou 8, **caractérisée en ce que** le plateau (4) présente une forme en coupe transversale sensiblement en forme de U avec une plaque supérieure (17) et des plaques latérales (18, 19) qui s'y raccordent des deux côtés, dans laquelle la plaque supérieure (17) présente des évidements (22) s'étendant d'une plaque latérale (18) à la plaque latérale opposée (19), dans laquelle respectivement un emballage primaire (1) est inséré par complémentarité de formes avec dans celui-ci un module de filtre (2) emballé.

10. Unité d'emballage stérile d'un module de filtre médical selon l'une quelconque des revendications 7 à 9, **caractérisée en ce que** le plateau (4) ou une pluralité de plateaux (4) peuvent être insérées ou sont insérées au moins partiellement par complémentarité de formes dans le suremballage (3).

11. Unité d'emballage stérile d'un module de filtre médical selon l'une quelconque des revendications précédentes, **caractérisée en ce que** le getter (14) induit une pression négative par rapport à la pression atmosphérique en raison de l'absorption de l'oxygène moléculaire de l'emballage primaire (1), dans laquelle la partie inférieure (6) conserve sensiblement sa forme, de sorte qu'uniquement le film supérieur (7) est déformé en raison de la pression négative.
